# EUROPEAN PATENT APPLICATION

(11) **EP 0 898 964 A1**
(43) Date of publication of application: **03.03.1999**
(21) Application number: 98306188.8
(22) Date of filing: 04.08.1998
(51) Int. Cl.: A61K 31/18

(54) **Method for treating heart failure**

(30) Priority: 19.08.1997 US 56149 P
(71) Applicant: PFIZER INC., New York, N.Y. 10017 (US)
(72) Inventor: Aurup, Peter, Belle Mead, New Jersey 08502 (US); Friedrich, Tilman, Groton Long Point, Connecticut 06340 (US); Hilton, Christopher John, Sandwich, Kent CT13 9NJ (GB); Marchant, Bradley Gerald, Sandwich, Kent CT13 9NJ (GB)
(74) Representative: Hayles, James Richard

(57) **Abstract**

A method for treating a mammal which presents with heart failure comprising administering to a mammal having heart failure a therapeutically effective amount of dofetilide or a pharmaceutically acceptable salt thereof.

## Description

### BACKGROUND OF INVENTION

This invention relates to the anti-arrhythmic agent dofetilide and its use in treating congestive heart failure (CHF).

The impact of heart failure continues to increase. Approximately three percent of the adult U.S. population (three to four million patients) have heart failure. With a steadily aging population, four hundred thousand individuals experience new onset heart failure each year, with a five year mortality rate approaching fifty percent. In 1991, alone, 2,280,445 patients were discharged from non-federal U.S. hospitals with a diagnosis of heart failure.

Congestive heart failure, regardless of its etiology, is characterized by a weakness of the myocardial tissue of the left and/or right ventricles of the heart to pump and circulate blood into systemic and/or pulmonary circulations. It is accompanied by circulatory and neurohumoral changes which result in failure to deliver sufficient blood and oxygen supply to peripheral tissues and vital organs. If left untreated the health of a patient with congestive heart failure could deteriorate to the point where the disease would be fatal. Congestive heart failure may be expressed as shortness of breath either on exertion, at rest or paroxysmal noctumal dyspnoea.

Although angiotensin converting enzyme (ACE) inhibitors are the only agents which have been shown to demonstrate a significant reduction in both hospitalization and mortality in heart failure patients, diuretics and digoxin continue to be more highly prescribed agents most likely due to their ability to provide effective symptomatic relief. However, digoxin's use is limited because of its slow onset of action and the small difference between the maximum therapeutic and minimum toxic dose levels. Moreover, the improved survival in patients treated with ACE inhibitors occurs primarily in the initial twelve months of therapy. Thus, a truly dramatic impact on disease progression has yet to be realized.

Dofetilide is a selective inhibitor of the rapid component of the delayed rectifier potassium current which prolongs the action potential duration and the effective refractory period in a concentration dependent manner. Clinical studies have demonstrated that dofetilide is effective in treating patients with atrial as well as ventricular arrhythmias. Specifically, for example, dofetilide has been studied in patients with severe left ventricular dysfunction (William M. Bailey et al., Electrophysiologic and Hemodynamic Effects of Dofetilide in Patients with Severe Left Ventricular Dysfunction, Circulation ,1992, Vol. 86, Part 4, Supplement 1, page 1265) in which it was stated that, unlike class I agents, dofetilide is safe in patients with congestive heart failure.

Thus, although there are a variety of treatments for congestive heart failure there is a continuing need and a continuing search in this field of art for altemative therapies.

### SUMMARY OF THE INVENTION

This invention is directed to a method for treating mammals which present with heart failure comprising administering to a mammal having heart failure therapeutically effective amount of dofetilide or a pharmaceutically acceptable salt thereof.

In a preferred aspect of this invention dofetilide is used.

In one particular aspect of this invention the mammal is a female or male human.

Preferably the amount of dofetilide is about 0.1 mg/day to about 5 mg/day. It is especially preferred that the amount of dofetilide is about 0.1 mg/day to about 2 mg/day.

A particular dosage of dofetilide is about 0.5 mg/day b.i.d.

In one aspect of this invention the heart failure is congestive heart failure.

Preferably the congestive heart failure does not worsen.

Dofetilide has Formula I below and may be named as methanesulfonamide, N-[4-[2-[methyl[2-[4-(methylsulfonyl)amino]phenoxy]ethyl]amino]phenyl]- or β-[(p-Methanesulfonamidophenethyl)methylamino]methanesulfono-p-phenetidide.

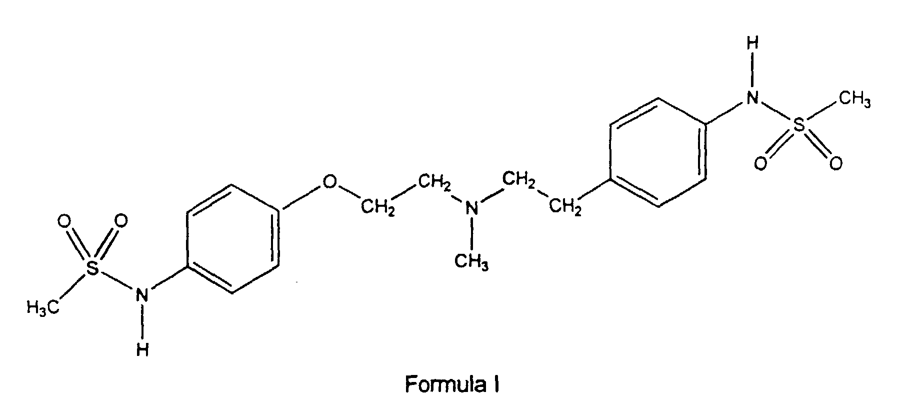

Heart failure refers to conditions in which the heart is no longer able to pump an adequate supply of blood in relation to the venous retum and in relation to the metabolic needs of the tissues of the body at that particular moment.

Congestive heart failure refers to that state in which abnormal circulatory congestion occurs as the result of heart failure. This includes but is not limited to circulatory failure due to mechanical abnormalities, myocardial (muscular) failure, peripheral circulatory failure and cardiac and noncardiac circulatory overload.

The term "treating", "treat" or "treatment" as used herein includes preventative (e.g., prophylactic) and palliative treatment.

By "pharmaceutically acceptable" it is meant the carrier, diluent, excipients, and/or salt must be compatible with the other ingredients of the formulation, and not deleterious to the recipient thereof.

The expression "pharmaceutically-acceptable salt" refers to nontoxic anionic salts containing anions such as (but not limited to) chloride, bromide, iodide, sulfate, bisulfate, phosphate, acetate, maleate, fumarate, oxalate, lactate, tartrate, citrate, gluconate, methanesulfonate and 4-toluene-sulfonate.

As used herein, the expressions "reaction-inert solvent" and "inert solvent" refers to a solvent which does not interact with starting materials, reagents, intermediates or products in a manner which adversely affects the yield of the desired product

Other features and advantages will be apparent from the specification and claims which describe the invention.

### DETAILED DESCRIPTION OF THE INVENTION

As mentioned above dofetilide is known in the art. Dofetilide is claimed and its preparation is described in U.S. Pat. No. 4,959,366, the disclosure of which is hereby incorporated by reference. The following description is provided as an aid to the preparation of dofetilide.

According to U.S. Pat No. 4,959,366 a solution of 1-(4-aminophenoxy)-2-[N-(4-aminophenethyl)-N-methylamino]ethane (0.75 g) and methanesulfonic anhydride (1.0 g) in dry methylene chloride (50 ml) was stirred at room temperature ovemight. After evaporation, the resultant oil was partitioned between a 2N aqueous sodium bicarbonate solution and ethyl acetate. After two further extractions with ethyl acetate, the organic portions were combined, dried over magnesium sulphate, filtered and evaporated. The resultant colorless solid (1.2 g) was crystallized from ethyl acetate/methanol to give dofetilide. 1-(4-aminophenoxy)-2-[N-(4-aminophenethyl)-N-methylamino]ethane was prepared from a solution of 1-(4-nitrophenoxy)-2-[N-methyl-N-(4-nitrophenethyl)amino]ethane (1.5 g) in ethanol (100ml). The solution was stirred at room temperature under three atmospheres of hydrogen in the presence of Raney nickel. The reaction mixture was filtered and evaporated to dryness. The residual oil was re-dissolved in ether, filtered and evaporated to give a yellow solid (1.1 g), which was crystallized from ethyl acetate/60°C-80°C petroleum ether to give the desired product, (0.9g), m.p. 73-74°C.

The starting materials and reagents for the above described compounds, are also readily available or can be easily synthesized by those skilled in the art using conventional methods of organic synthesis.

The compound of this invention is basic and it forms salts with pharmaceutically acceptable anions. All such salts are within the scope of this invention and they can be prepared by conventional methods. For example, they can be prepared simply by contacting the acidic and basic entities, usually in a stoichiometric ratio, in either an aqueous, non-aqueous or partially aqueous medium, as appropriate. The salts are recovered either by filtration, by precipitation with a nonsolvent followed by filtration, by evaporation of the solvent, or, in the case of aqueous solutions, by lyophilization, as appropriate.

The utility of the compound of the present invention as a medical agent in the treatment of heart failure in mammals (e.g. humans) is demonstrated by the activity of the compound of this invention in conventional assays and the clinical protocol described below. Such assays and clinical protocol also provide a means whereby the activities of the compound of this invention can be compared with the activities of other known compounds. The results of these comparisons are useful for determining dosage levels in mammals, including humans, for the treatment of such diseases.

The compound of this invention is readily adapted to clinical use as a heart failure treating agent.

A more detailed protocol is described immediately hereinafter.

The design of the Danish Investigations of Arrhythmia and Mortality on

Dofetilide (DIAMOND-CHF) study has been described (Clinical Cardiology Dofetilide in Patients with Left Ventricular Dysfunction and either Heart Failure or Acute Myocardial Infarction: Rationale, Design and Patient Characteristics of the Diamond studies (The DIAMOND Study Group, Clin. Cardiol. 20, 706-710(1997)). In brief, it is a randomized double blind, placebo-controlled, parallel group study conducted on a multicentre basis at 34 hospitals in Denmark. It was required that consecutive patients hospitalized with CHF were screened for entry. CHF was defined as recent (within one month) shortness of breath either at rest or upon minimal exertion or paroxysmal noctumal dyspnoea. When patients were screened, an echocardiography was recorded on videotape and evaluated in a central laboratory. Wall motion Index was evaluated using a 16 segment model of the left ventricle (Schiller NB, Shah PM, Crawford M, DeMaria A, Devereux R, Feigenbaum H, et al. Recommendations for Quantitation of the Left Ventricle by Two-Dimensional Echocardiography. J American Society of Echocardiography 1989; 2:358-367).

Patients were eligible according to left ventricular systolic function if wall motion index was less than or equal to 1.2 (approximately corresponding to an ejection fraction of < 35%). Other inclusion criteria were: hospitalization with CHF/development of CHF within 3-7 days, age ≥ 18 years, non-child bearing potential. Exclusion criteria were limited to those required for safety reasons- Heart rate less than 50 beats/min when awake; sinoatrial block or 2-3 degree atrioventricular block not treated with a pacemaker, history of drug induced proarrhythmia; QTc(QTc = QT/ RR^{½} interval measured in milli seconds) interval exceeding 460 msec (500 msec in case of bundle branch block); diastolic blood pressure > 115 mm Hg or systolic blood pressure less than 80 mm Hg; patients who were likely to die from other causes during the course of the study; serum potassium less than 3.6 mmol/L or >5.5 mmol/L; concurrent therapy with Class I or III antiarrhythmic drugs; amidarone treatment within the last 3 months; participation in experimental drug studies in the previous 3 months; previous treatment with dofetilide; inability to co-operate in the study; creatinine clearance (Cockroft's formula) < 20 ml/min (Cockroft et al. ( Nephron(1976);16:31-41)); significant liver dysfunction; acute myocarditis; planned cardiac surgery or angioplasty; aortic stenosis; cardiac operation within the preceding 4 weeks; implanted Cardioverter Defibrillator.

Eligible patients were assigned to treatment by means of a computer generated pseudo random code and patients were stratified according to center and degree of LV dysfunction (wall motion index < 0.8 and wall motion index ≥ 0.8). At randomization the patients were monitored by continuous telemetry in hospital for the first 72 hours of study treatment to ensure recognition of possible proarrhythmic events. When feasible Holter monitoring was performed on the day prior to randomization and on the third day of treatment.

Patients in sinus rhythm and creatinine clearance (Cockroft's formula) > 60 ml/min received dofetilide/placebo 0.5 mg twice daily. If creatinine clearance was less than 60 ml/min and greater than or equal to 40 ml/min or the patient had atrial fibrillation the dose was 0.25 mg twice daily. If creatinine clearance was less than 40 and greater than or equal to 20 ml/min the dose was 0.25 mg once daily. With the higher doses, stepwise dose reduction was made if creatinine clearance decreased, at the discretion of the investigator or if there was an increase in QTc interval exceeding 20% from baseline or exceeding 550 msec. If QTc did not fall below an acceptable limit within 2 days further dose reduction was performed. If a dose reduction was required at the lowest possible dose study medication was discontinued.

Clinical evaluation, laboratory safety tests and adverse events were recorded and a 12-lead ECG including measurement of QTc was obtained during hospitalization, after 1 month and subsequently every 3 months after initiation of therapy. After 1 year of treatment, laboratory safety tests were obtained on a 6 monthly basis. Follow up of survival status during the study and at study closure was completed using the Danish Central Person Register where all deaths in the country are registered within 2 weeks.

The primary endpoint was death from all causes. Prespecified secondary endpoints were Cardiac mortality; Total Arrhythmic Death (TAD); cardiac mortality plus resuscitated cardiac arrest; worsening CHF; number of infarctions/reinfarctions; arrhythmia requiring treatment and withdrawal of study drug. In patients with atrial fibrillation at baseline, separate analysis of total mortality/number of strokes/number of systemic embolisms were performed. Analysis of mortality endpoints was repeated on patients randomized from the time of the implementation of the protocol amendment allowing for dosing according to the creatinine clearance. The Event Committee dassified death as cardiac if there was no specific evidence that death was noncardiac. Presumed arrhythmia death was defined according to the CAST criteria (Greene HL, Richardson DW, Barker AH, Roden DM, Capone RJ, Echt DS, et al. Classification of deaths after myocardial infarction as arrhythmic or nonarrhythmic (the Cardiac Arrhythmia Pilot Study). Am J Cardiol 1989; 63:1-6.) with the exception that resuscitated cardiac arrest was not counted as death. Documented arrhythmic death required that the fatal arrhythmia had been documented with an ECG. Total arrhythmic death was defined as presumed or documented arrhythmic death. The secondary endpoint, worsening of heart failure, was documented by the investigator and required both hospital admission with heart failure and an increase in drug treatment.

The data was analyzed using conventional methods appropriate to the data, for example, Kapalyn-Meier methods and compared between treatments using the log rank test. The relative risk (dofetilide to placebo) once obtained using Cox's proportional hazards model.

A total of 5548 patients admitted with CHF were screened for entry into the study. As patients could be screened more than once 5812 screenings were performed. Of these 2933 (53%) were eligible according to their having reduced left ventricular systolic function. Either due to exclusion criteria or lack of informed consent, 1415 patients were excluded, leaving 1518 patients randomized (26% of all screened). Baseline characteristics of the two treatment groups were similar. At discharge from hospital, 424 patients (187 on dofetilide, 237 on placebo) were allocated to a dose of 1 mg daily, 628 patients (338 on dofetilide, 290 on placebo) to 0.5 mg daily, and 316 patients (154 on dofetilide, 162 on placebo) to 0.25 mg daily. Slightly more patients on dofetilide were maintained on trial medication. At 1 year, 421 (55%) patients were continuing on dofetilide and 397 (53%) on placebo. The median duration of therapy was 383 days in the dofetilide group and 371 days in the placebo group. The study was continued as scheduled until 1 year after indusion of the last patient.

A total of 628 patients in the study died, 311 in the dofetilide group and 317 in the placebo group. Survival in the two groups- did not differ significantly, and the overall risk reduction on dofetilide compared with placebo was 0.954 (95% confidence limits 0.81-1.11. p=0.53) When comparisons of survival were made in patients maintained on trial medication the 1 year survival rate in the dofetilide and placebo groups were 89% and 89%, respectively, and when compared over the entire duration of the study there was no significant difference between treatments (p=0.54)

There was a significant increase in the time to hospitalization with worsening heart failure (i.e., reduction in worsening heart failure) for the dofetilide group compared with placebo. A total of 229 (30.0%) patients on dofetilide and 290 (38.4%) on placebo developed worsening heart failure (p less than 0.001^{∗}). [^{∗}P values refer to the time to first event analysis and relate to the entire duration of the study]

Dofetilide is known to be effective in converting patients with atrial fibrillation to sinus rhythm and maintaining sinus rhythm in the long term. This has been confirmed in the above study where patients with atrial fibrillation at baseline more often converted to sinus rhythm with or without electrical cardioversion (141/190 on dofetilide and 106/201 on placebo; p <0.001^{∗}) and patients with sinus rhythm at baseline less often developed atrial fibrillation (12/570 on dofetilide and 40/553 on placebo; p<0.001^{∗}).

Patients with atrial fibrillation who convert to sinus rhythm may be expected to have an improvement in cardiac function and a reduced need for hospitalization due to the improved hemodynamics associated with sinus rhythm. In the above study, 391 patients (25.8%) had atrial fibrillation or flutter at entry to the study and the benefit seen in heart failure could be due to the effect of dofetilide on rhythm in these patients. In order to determine if this was the case, the endpoint of hospitalization for worsening CHF was re-examined with those patients with atrial fibrillation or flutter at baseline excluded and, in addition, patients who developed atrial fibrillation or flutter during the study were censored at the last time point that they were in sinus rhythm. This analysis allows the examination of the endpoint of worsening CHF with all influence of atrial fibrillation removed and showed that the benefit in reduction in worsening heart failure was still present in these patients (169/570 on dofetilide and 189/553 on placebo; p=0.029^{∗}).

Since the time to hospitalization with worsening heart failure on dofetilide was still increased when the influence of atrial fibrillation was excluded, the improvement cannot be due to the beneficial effect on rhythm alone.

In conclusion, a highly significant reduction in worsening congestive heart failure for dofetilide was found (independent of its effects as an antiarrythmic agent) and, in addition, mortality in patients with congestive heart failure and left ventricular dysfunction was neither increased nor decreased by treatment with dofetilide.

Generally, the compound of this invention is administered orally, but parenteral administration (e.g., intravenous, intramuscular, subcutaneous or intramedullary) may be utilized, for example, where oral administration is inappropriate for the instant target or where the patient is unable to ingest the drug. Topical administration may also be indicated, for example, where the patient is suffering from gastrointestinal disorders or whenever the medication is best applied to the surface of a tissue or organ as determined by the attending physician.

An amount of dofetilide that is effective for the heart failure treatment of this invention, for example congestive heart failure treatment is used. Typically, an effective dosage for dofetilide is in the range of about 0.1 mg/day to 5 mg/day in single or divided doses, preferably 0.1 mg/day to 2 mg/day in single or divided doses. It is especially preferred that the dosage is about 0.5 mg b.i.d..

In certain patients (described below) the dosage is preferably modified as described.

In those patients who exhibit atrial fibrillation or flutter 0.25 to 0.5 mg b.i.d. dofetilide is used.

It is believed that body weight, gender and renal function may influence the risk of Torsades de Pointes and that renal function may influence exposure to dofetilide. Thus, for renally impaired patients the dosage of dofetilide is preferably amended by adjusting the dofetilide starting dose based on creatinine clearance (Clcr) estimated from serum creatinine using the Cockroft Gault formula. This adjustment aims to normalize individual exposure to dofetilide. This is described more fully below.

In those patients who have a creatinine clearance calculated on the basis of either the equation by Cockroft et al. ( Nephron(1976);16:31-41) or the nomogram by Kampmann J. et al. (Acta Med Scand (1974);196:517-20) of between 40 and 60 ml/min, 0.25mg b.i.d. dofetilide is used.

In those patients who have a creatinine clearance calculated on the basis of either the equation by Cockroft et al. or the nomogram by Kampmann J. et al of between 40 and 20 ml/min, 0.25mg o.d. dofetilide is used.

| | CrCl≥60ml/min | 40≤CrCl<60ml/min | 20≤CrCl<40ml,min |
|---|---|---|---|
| -AF/AFL | 0.50 mg b.i.d. | 0.25 mg b.i.d. | 0.25 mg o.d. |
| +AF/AFL | 0.25 mg b.i.d. | 0.25 mg b.i.d. | 0.25 mg o.d. |
| -AF/AFL means no presence of atrial fibrilation or atrial flutter. | | | |
| +AF/AFL means that there is atrial fibrilation or atrial flutter present. | | | |

Adjustments to the dosage may be made on the basis of changes to the QTc interval, the occurrence of either subjective adverse events or atrial fibrillation or a decrease in creatinine clearance.

In any event, the amount and timing of compound administered will, of course, be dependent on the subject being treated, on the severity of the affliction, on the manner of administration and on the judgment of the prescribing physician. Thus, because of patient to patient variability, the dosages given above are a guideline and the physician may titrate doses of dofetilide to achieve the treatment (e.g., congestive heart failure improvement) that the physician considers appropriate for the patient. In considering the degree of treatment desired, the physician must balance a variety of factors such as age of the patient, presence of preexisting disease, as well as presence of other diseases (e.g., diabetes).

Dofetilide may be administered alone or in combination with digitalis, thiazide diuretics, other diuretics, ACE inhibitors, etc.

The compound of the present invention is generally administered in the form of a pharmaceutical composition comprising dofetilide together with a pharmaceutically acceptable vehicle or diluent. Thus, dofetilide can be administered individually or together in any conventional oral, parenteral, rectal or transdermal dosage form.

For oral administration a pharmaceutical composition can take the form of solutions, suspensions, tablets, pills, capsules, powders, and the like. Tablets containing various excipients such as so.d.ium citrate, calcium carbonate and calcium phosphate are employed along with various disintegrants such as starch and preferably potato or tapioca starch and certain complex silicates, together with binding agents such as polyvinylpyrrolidone, sucrose, gelatin and acacia. Additionally, lubricating agents such as magnesium stearate, so.d.ium lauryl sulfate and talc are often very useful for tabletting purposes. Solid compositions of a similar type are also employed as fillers in soft and hard-filled gelatin capsules; preferred materials in this connection also include lactose or milk sugar as well as high molecular weight polyethylene glycols. When aqueous suspensions and/or elixirs are desired for oral administration, the compounds of this invention can be combined with various sweetening agents, flavoring agents, coloring agents, emulsifying agents and/or suspending agents, as well as such diluents as water, ethanol, propylene glycol, glycerin and various like combinations thereof.

For purposes of parenteral administration, solutions in sesame or peanut oil or in aqueous propylene glycol can be employed, as well as sterile aqueous solutions of the corresponding water-soluble salts. Such aqueous solutions may be suitably buffered, if necessary, and the liquid diluent first rendered isotonic with sufficient saline or glucose. These aqueous solutions are especially suitable for intravenous, intramuscular, subcutaneous and intraperitoneal injection purposes. In this connection, the sterile aqueous media employed are all readily obtainable by standard techniques well-known to those skilled in the art.

For purposes of transdermal (e.g., topical) administration, dilute sterile, aqueous or partially aqueous solutions (usually in about 0.1% to 5% concentration), otherwise similar to the above parenteral solutions, are prepared.

Methods of preparing various pharmaceutical compositions with a certain amount of active ingredient are known, or will be apparent in light of this disclosure, to those skilled in this art. For examples of methods of preparing pharmaceutical compositions, see Remington's Pharmaceutical Sciences, Mack Publishing Company, Easter, Pa., 15th Edition (1975).

Pharmaceutical compositions according to the invention may contain 0.1%-95% of the compound of this invention, preferably 1%-70%. In any event, the composition or formulation to be administered will contain a quantity of a compound according to the invention in an amount effective to treat the disease/condition of the subject being treated, e.g., congestive heart failure.

## Claims

1. Use of dofetilide, or a pharmaceutically acceptable salt thereof, in the manufacture of a medicament for the treatment of heart failure in a mammal.

2. The use as claimed in claim 1, wherein dofetilide is used.

3. The use as claimed in claim 1 or claim 2, wherein the mammal is a female or male human.

4. The use as claimed in any one of the preceding claims, wherein the amount of dofetilide, or a pharmaceutically acceptable salt thereof, administered is about O.1mg/day to about 5mg/day.

5. The use as claimed in claim 4, wherein the amount of dofetilide, or a pharmaceutically acceptable salt thereof, administered is about 0,1mg/day to about 2mg/day.

6. The use as claimed in claim 5, wherein the amount of dofetilide, or a pharmaceutically acceptable salt thereof administered is about 0.5mg/day b.i.d..

7. The use as claimed in any one of the preceding claims, wherein the heart failure is congestive heart failure.

8. The use as claimed in claim 7, wherein the congestive heart failure does not worsen.
